# EUROPÄISCHE PATENTSCHRIFT

(11) **EP 2 027 843 B1**
(45) Veröffentlichungstag und Bekanntmachung des Hinweises auf die Patenterteilung: **28.03.2012**
(21) Anmeldenummer: 08020127.0
(22) Anmeldetag: 10.06.2005
(51) Int. Cl.: A61F 13/15

(54) **Wegwerfbarer absorbierender Hygieneartikel in Pantform**
Disposable absorbent hygiene article in pant form
Article hygiénique absorbant jetable en forme de culotte

(30) Priorität: 29.06.2004 DE 102004032377
(43) Veröffentlichungstag der Anmeldung: 25.02.2009
(62) Teilanmeldung aus: 07013345.9
(73) Patentinhaber: Paul Hartmann AG, 89522 Heidenheim (DE)
(72) Erfinder: Bittner, Manuela, 89522 Heidenheim (DE); Jovanov, Sascha, 89522 Heidenheim (DE); Hornung, Fridmann, Dr, Penalolén, Santiago (CL)
(74) Vertreter: Friz, Oliver

(56) Entgegenhaltungen:
- WO-A-01/85082
- US-A- 5 735 839

## Beschreibung

Die Erfindung betrifft einen wegwerfbaren absorbierenden Hygieneartikel in Pantform, mit einem eine Hüftöffnung bildenden in Umfangsrichtung durchgehend geschlossenen Hüftrand und mit Beinöffnungen, wobei der in Umfangsrichtung durchgehend geschlossene Hüftrand und die Beinöffnungen gebildet sind, indem Längsseitenrandabschnitte eines Vorderteils und eines Rückenteils herstellerseitig miteinander verbunden sind, und mit einem Absorptionskörper und mit Elastifizierungsmitteln zumindest im Bereich der Beinöffnungen.

Derartige absorbierende Hygieneartikel in Höschenform sind bekannt. Sie umfassen zumeist eine Vielzahl von Elastifizierungsmitteln, häufig in Form von gummielastischen Fäden, die im vorgespannten Zustand mit Chassismaterialien zumeist klebend verbunden werden. Dabei ist üblicherweise ein Hüftrandbereich vorzugsweise durchgehend in Umfangsrichtung elastifiziert. Auch im Vorderbereich und im Rückenbereich sind bei bekannten Windelhosen Elastifizierungsmittel vorgesehen. Desgleichen werden die Beinöffnungen umgebende bzw. die Beinöffnungen bildende Umfangsbereiche durchgehend elastifiziert, damit dort ein weitgehend dichtendes Anliegen des Hygieneartikels an die Hautoberfläche des Benutzers gewährleistet ist, um das seitliche Austreten von Körperausscheidungen zu verhindern.

US 5 735 839 und WO 0185082 zeigen wegwerfbare absorbierende Hygieneartikel in Pantform.

Wenn unter Vorspannung stehende elastifizierte Bereiche gerade im Bereich der Beinöffnungen gegen die Hautoberfläche anliegen, so geht dies oftmals mit Hautreizungen einher, die durch die Reibung oder Krafteinwirkung von zumeist fadenförmigen Elastifizierungsmitteln hervorgerufen werden. Wenn zusätzlich feuchte Bedingungen innerhalb des Hygieneartikels durch Körperflüssigkeiten einerseits oder durch Schweißabsonderungen andererseits hinzukommen, so stellt sich dieses Problem in noch gravierenderem Maße. Es wird weiter durch zunehmende Aktivität, also Bewegungen des Benutzers, verschärft.

Hiervon ausgehend liegt der vorliegenden Erfindung die Aufgabe zugrunde, den Tragkomfort absorbierender Hygieneartikel der eingangs genannten Art zu verbessern und insbesondere das Auftreten von Hautirritationen zu verringern.

Diese Aufgabe wird bei einem gattungsgemäßen Hygieneartikel erfindungsgemäß dadurch gelöst, dass im flachgelegten Zustand vor der herstellerseitigen Verbindung der Längsseitenrandabschnitte ausgehend von einem Längsseitenrand des Vorderteils des Artikels entlang eines Umfangs einer jeweiligen Beinöffnung in Richtung auf eine Schrittmittellinie ein erster elastifizierter Umfangsabschnitt, daran anschließend ein zweiter nicht elastifizierter Umfangsabschnitt und daran anschließend ein dritter elastifizierter Umfangsabschnitt vorgesehen sind, dass im wesentlichen in einer Querrichtung erstreckte Elastifizierungsmittel im Schrittbereich vorgesehen sind und dass die Elastifizierungsmittel des dritten Umfangsabschnitts in die Elastifizierungsmittel des Schrittbereichs übergehen.

Es wurde erfindungsgemäß erkannt, dass nicht die gesamte Beinöffnung einer Windelhose elastifiziert zu werden braucht. Vor dem Hintergrund, dass moderne Hygieneartikel zusätzlich über im Wesentlichen in Längsrichtung verlaufende aufstehende Bündchenelemente, sogenannte "Cuff-Elemente" verfügen, die eine seitliche Barriere für das Austreten von Flüssigkeiten und auch festen Körperausscheidungen bilden, erscheint es als nicht mehr zwingend erforderlich, die Beinöffnungen in Umfangsrichtung durchgehend zu elastifizieren. Da Elastifizierungsmittel eine Raffung oder Rüschung von mit ihnen verbundenen Materialien bewirken, wenn sie im vorgespannten Zustand mit diesen Materialien verbunden werden, führen sie im relaxierten Zustand zu einer Materialanhäufung, die beispielsweise in sitzender Position auch als unangenehm empfunden werden kann. Jedenfalls reiben insbesondere bei Bewegung des Benutzers geraffte oder gerüschte Bereiche, also elastifizierte Bereiche, in besonderem Maße gegen die Hautoberfläche des Benutzers, was zu den vorausgehend erwähnten Hautirritationen führen kann. Es wurde erfindungsgemäß erkannt, dass dem dadurch entgegengewirkt werden kann, dass im Schrittbereich möglichst nur dort Elastifizierungsmittel eingesetzt werden, wo sie wirklich erforderlich sind. Dadurch, dass erfindungsgemäß zwischen dem ersten Umfangsabschnitt, welcher dem Hüftbereich zugewandt ist, und dem dritten Umfangsabschnitt, welcher zwischen den Beinen des Benutzers angeordnet ist, ein zweiter nicht elastifizierter Umfangsabschnitt zwischengeordnet ist und im Bereich einer Schrittmittellinie ein weiterer nicht elastifizierter vierter Umfangsabschnitt vorgesehen ist, wird ein besserer Tragkomfort erzielt. In diesem zweiten und vierten Umfangsabschnitt liegen die betreffenden Materialabschnitte des Hygieneartikels unter sanfter Vorspannung gegen die Hautoberfläche des Benutzers an. Da in dem zweiten Umfangsabschnitt keine sich stets verändernde Raffung oder Rüschung gebildet oder ausgedehnt wird, kommt es auch nicht zu Reibungen mit der Hautoberfläche. Außerdem wird ein Luftaustausch mit dem Inneren des Hygieneartikels ermöglicht und damit das Mikroklima in von dem Hygieneartikel bedeckten Bereichen der Hautoberfläche wesentlich verbessert.

Eine erfindungsgemäße Verbesserung wird bereits erreicht, wenn nur entlang des dem Vorderteil zugewandten Umfangsbereichs der beiden Beinöffnungen ein solcher nicht elastifizierter zweiter Umfangsabschnitt vorgesehen ist. In Weiterbildung dieses grundsätzlichen Erfindungsgedankens erweist es sich aber als vorteilhaft, wenn sowohl die Umfangsbereiche im Vorderteil als auch im Rückenteil in der erfindungsgemäßen Weise gestaltet sind, so dass je Beinöffnung wenigstens drei nicht elastifizierte Umfangsabschnitte vorgesehen sind.

Es erweist sich ferner als vorteilhaft, wenn im Wesentlichen in einer Querrichtung erstreckte Elastifizierungsmittel im Schrittbereich des Hygieneartikels vorgesehen sind. Ein solches Elastifizierungsmittel kann im Bereich des Absorptionskörpers zu einer gewollten Zusammenziehung des Saugkörpers im Schrittbereich führen. Es ist nämlich durchaus gewollt und vorteilhaft, dass der Absorptionskörper im Schrittbereich schmal ausgebildet ist, aber dennoch eine hinreichende Absorptionskapazität, also absorbierendes Material, zur Verfügung stellt. Durch eine Zusammenziehung kann dann das Flächengewicht in diesem Bereich erhöht werden, um in diesem Bereich eine große Absorptionskapazität zur Verfügung zu stellen. Außerdem führen im Schrittbereich in Querrichtung verlaufende Elastifizierungsmittel zu einer Formgebung des Hygieneartikels. Es wird in vorteilhafter Weise eine Schalenform im Schrittbereich erzeugt.

Das im Schrittbereich im Wesentlichen in Querrichtung verlaufende Elastifizierungsmittel oder die Elastifizierungsmittel könnten separat von denjenigen Elastifizierungsmitteln, welche die Beinöffnungen elastifizieren, gebildet sein. Nach einer weiteren Ausführungsform der Erfindung erweist es sich als vorteilhaft, dass die Elastifizierungsmittel des dritten Umfangsabschnitts in die Elastifizierungsmittel des Schrittbereichs übergehen, d. h. das oder die in Querrichtung verlaufenden Elastifizierungsmittel im Schrittbereich bilden, indem sie ausgehend von ihrer Erstreckung in Umfangsrichtung der Beinöffnungen dann im Wesentlichen in die Querrichtung übergehen. Sie können dabei in einem im Wesentlichen stetigen Kurvenverlauf oder auch unstetig abzweigen, wobei einem stetigen Verlauf der Vorzug zu geben ist. Wenn die Elastifizierungsmittel ausgehend von dem dritten Umfangsabschnitt in einer Kurve nach innen laufen und den Schrittbereich in Querrichtung traversieren, so kann dadurch der vierte, nicht elastifizierte Umfangsabschnitt gebildet werden. Vorzugsweise überfängt der vierte, nicht elastifizierte Umfangsabschnitt die Schrittmittellinie. Die Schrittmittellinie verläuft dabei durch das Minimum der Schrittbereichsbreite des Hygieneartikels. Sollte das Minimum nicht eindeutig bestimmbar sein, so verläuft die Schrittmittellinie durch das Zentrum des Schrittbereichs des Hygieneartikels. Das Zentrum des Schrittbereichs eines Hygieneartikels lässt sich bestimmen, indem bei einem aufrechtstehenden Benutzer oder einem langgestreckt auf eine ebene Unterlage gelegten Benutzer ein elastischer Faden oder ein Gummiband achtförmig um die Beine herumgelegt wird, so dass ein Kreuzungspunkt des Fadens oder Bands zwischen den Beinen entsteht. Dieser Kreuzungspunkt wird als Zentrum des Schrittbereichs des Hygieneartikels definiert, wenn dieser bestimmungsgemäß getragen wird.

In dem jeweiligen nicht elastifizierten zweiten und/oder vierten Umfangsabschnitt könnten einfach keine Elastifizierungsmittel vorgesehen werden. Es wäre aber auch denkbar und vorteilhaft, wenn im Zuge der Herstellung des erfindungsgemäßen Hygieneartikels das oder die Elastifizierungsmittel zunächst in Umfangsrichtung der Beinöffnungen durchgehend erstreckt wird bzw. werden und dann der nicht elastifizierte zweite und/oder vierte Umfangsabschnitt dadurch erhalten wird, dass dort zuvor erstreckte Elastifizierungsmittel durchtrennt werden. Die Elastifizierungsmittel werden dann relaxieren bzw. zurückschnellen, soweit sie in diesem zweiten und/oder vierten Umfangsabschnitt nicht mit Chassis bildenden Materialien z. B. klebend verbunden werden. Eine solche Verbindung der Elastifizierungsmittel sollte dann auf den ersten und dritten Umfangsabschnitt, der ja elastifiziert werden soll, beschränkt werden, damit die durchtrennten Elastifizierungsmittel in dem zweiten Umfangsabschnitt relaxieren können und somit ihre elastifizierende Wirkung auf diesen zweiten Umfangsabschnitt verlieren.

Die Durchtrennung der Elastifizierungsmittel in dem zweiten und/oder vierten Umfangsabschnitt kann beispielsweise durch einen einzigen Schnitt erfolgen. Es wäre aber auch denkbar und vorteilhaft, dass die Durchtrennung der Elastifizierungsmittel durch eine Vielzahl von Schnitten erfolgt, so dass die in dem zweiten und/oder vierten Umfangsabschnitt zuvor durchgehend erstreckten Elastifizierungsmittel in eine Vielzahl von kleinen Abschnitten, insbesondere einer Länge im Millimeterbereich, geteilt werden und so ihre den zweiten und/oder vierten Umfangsabschnitt elastifizierende Wirkung verlieren. Solchenfalls können die Elastifizierungsmittel auch im zweiten und/oder vierten Umfangsabschnitt z. B. klebend mit Chassismaterialien verbunden sein, ohne dass dies die Relaxierung behindern würde. Ein einfaches oder mehrfaches Durchtrennen der Elastifizierungsmittel kann auch mittels Lasertechnik erfolgen.

Nach einer weiteren bevorzugten Ausführungsform der Erfindung ist der nicht elastifizierte zweite und/oder vierte Umfangsabschnitt dadurch erhalten, dass dort zuvor erstreckte Elastifizierungsmittel durch Anwendung von Hitze und/oder Druck und/oder Lasereinwirkung ihrer elastifizierenden Wirkung benommen worden sind. Insbesondere kann dies mittels Beaufschlagung durch Ultraschall erreicht werden. Auch auf diese Weise kann ein dort zuvor erstrecktes unter Vorspannung stehendes Elastifizierungsmittel inaktiviert werden, indem es durch insbesondere punktuelle oder rasterartige Anwendung von Druck und/oder Temperatur, insbesondere Ultraschall, derart verformt und insbesondere auch durchtrennt wird, dass zumindest seine den jeweiligen Umfangsabschnitt elastifizierende Wirkung in Wegfall gerät.

Die Anwendung von Hitze und/oder Druck bringt gegenüber der Anbringung von Trennschnitten den Vorteil mit sich, dass hierbei weniger die Gefahr besteht, dass Chassis bildende Materialien in ungewollter Weise mitgeschnitten oder perforiert werden. Auf der anderen Seite kann es sich als besonders vorteilhaft erweisen, dass gerade im Bereich des zweiten und/oder vierten Umfangsabschnitts die Chassis bildenden Materialien durchtrennt oder perforiert werden, um dort zusätzlich eine Atmungsaktivität des Hygieneartikels bewusst zur Verfügung zu stellen. Insbesondere wenn Absorptionskörper in Form einer funktionellen Einheit mit einer absorbierenden Struktur und einem flüssigkeitsundurchlässigen Backsheet unmittelbar unterhalb der absorbierenden Struktur zum Einsatz kommen, ist eine Flüssigkeitsundurchlässigkeit der übrigen Chassismaterialien nämlich nicht zwingend erforderlich, sondern es kann sogar erwünscht sein, dass zur Erreichung einer Atmungsaktivität nur luftdurchlässige Chassismaterialien verwendet werden. Bei Verwendung vorgenannter funktioneller Einheiten als Absorptionskörper können dann in vorteilhafter Weise Chassismaterialien aus Vliesstoffen oder Vliesstofflaminaten eingesetzt werden; auch Vliesfolienlaminate mit atmungsaktiv oder sogar flüssigkeitsdurchlässig ausgebildeten Folien sind denkbar. Vorgenannte funktionelle Einheiten als Absorptionskörper können auch integrierte Topsheet-Lagen aufweisen und in vorteilhafter Weise aufstehende elastifizierte Bündchenelemente (sogenannte "Cuffs"), welche eine seitliche Auslaufsperre bilden. Solche Bündchenelemente können aber auch außerhalb des Absorptionskörpers oder der funktionellen Einheit als Absorptionskörper vorgesehen sein.

Nach einem weiteren Erfindungsgedanken erweist es sich als vorteilhaft, wenn eine in Querrichtung durchgehende Spur einer Anwendung von Hitze und/oder Druck vorgesehen ist, welche auch den zweiten und/oder vierten Umfangsabschnitt bei der jeweiligen Beinöffnung überfängt. Diese in Querrichtung durchgehende Spur kann dann in vorteilhafter Weise durch eine in Maschinenrichtung durchgehende, also ununterbrochene Bearbeitung der den Hygieneartikel bildenden Materialien erhalten werden, was sich in herstellungstechnischer Hinsicht als vorteilhaft erweist.

Zur Offenbarung einer Technik der Anwendung von Hitze und/oder Druck zur Inaktivierung von Elastifizierungsmitteln wird auf EP 1 374 814 A1 verwiesen.

Es hat sich als zweckmäßig und vorteilhaft erwiesen, wenn die Erstreckung des ersten elastifizierten Umfangsabschnitts in Umfangsrichtung der Beinöffnungen am besten betrachtet im flachgelegten Zustand der den Hygieneartikel bildenden Flachmaterialien 3 bis 15 cm, insbesondere 4 bis 15 cm, insbesondere 5 bis 15 cm und weiter insbesondere 5 bis 13 cm beträgt. Der flachgelegte Zustand bedeutet dabei, dass der Hygieneartikel derart auf eine Unterlage aufgebracht oder projeziert ist, dass seine Chassismaterialien nicht mehr durch die Elastifizierungsmittel gerafft sind. Entsprechend der oben beschriebenen Lage dieses ersten Umfangsabschnitts erstreckt er sich bei Betrachtung des fertig konfigurierten Hygieneartikels im angelegten Zustand an einen Benutzer von der Seite vom hüftzugewandten Scheitel der jeweiligen Beinöffnung ausgehend in Uhrzeigersinnrichtung oder entgegen der Uhrzeigersinnrichtung.

Die Erstreckung des zweiten nicht elastifizierten Umfangsabschnitts in Umfangsrichtung im flachgelegten Zustand beträgt vorteilhafterweise 1 bis 10 cm, insbesondere 2 bis 8 cm und weiter insbesondere 3 bis 8 cm.

Die Erstreckung des dritten wiederum elastifizierten Umfangsabschnitts in Umfangsrichtung im flachgelegten Zustand beträgt vorteilhafterweise 1 bis 8 cm, insbesondere 1 bis 7 cm und weiter insbesondere 1 bis 5 cm.

Die Erstreckung des vierten nicht elastifizierten Umfangsabschnitts in Umfangsrichtung im flachgelegten Zustand beträgt vorteilhafterweise 1 bis 7 cm, insbesondere 1 bis 5 cm und weiter insbesondere 1 bis 4 cm.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform des erfindungsgemäßen Hygieneartikels. In der Zeichnung zeigt:
- Figur 1: eine Draufsicht auf eine erste Ausführungsform eines erfindungsgemäßen Hygieneartikels im flachgelegten Zustand vor der herstellerseitigen Verbindung von Längsseitenrandbereichen;
- Figur 2: eine Figur 1 entsprechende Darstellung einer weiteren Ausführungsform eines erfindungsgemäßen Hygieneartikels.

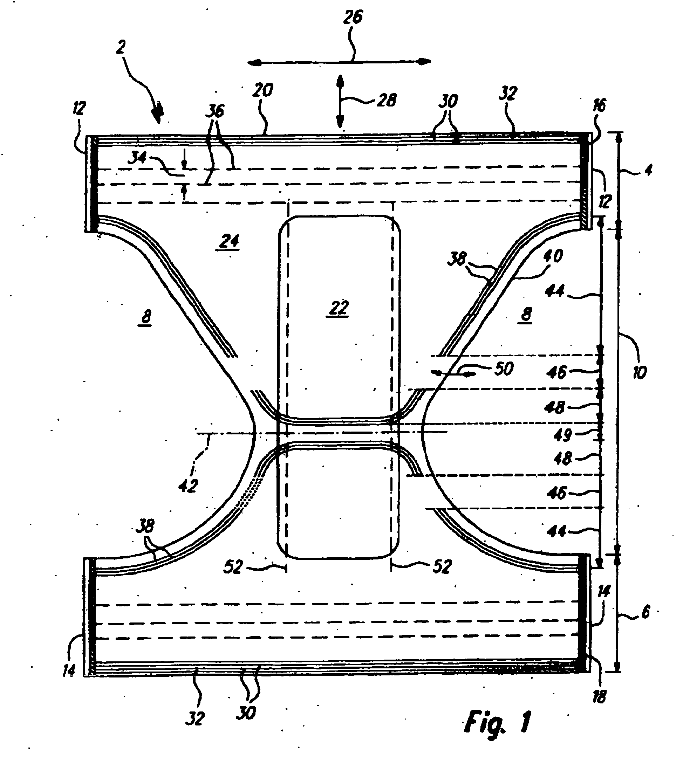

Figur 1 zeigt einen insgesamt mit dem Bezugszeichen 2 bezeichneten Hygieneartikel in Form einer Windelhose ("pant"), jedoch im flachgelegten Zustand vor der herstellerseitigen Verbindung der Längsseitenrandabschnitte. Der Hygieneartikel umfasst einen Rückenteil 4, einen Vorderteil 6 und einen dazwischen liegenden, die späteren Beinausschnitte 8 begrenzenden Schrittteil 10. Im flachgelegten Zustand wird der Rückenteil 4 von Längsseitenrändern 14 begrenzt. Diese werden zur Bildung der Pant-Form im Bereich der schraffierten Zonen 16 bzw. 18 miteinander verbunden, etwa durch Heißsiegeln oder sonstige übliche Fügeverfahren. Auf diese Weise werden zwei Seitennahtbereiche des Hygieneartikels gebildet, die nicht zerstörungsfrei gelöst werden können. Der fertig konfigurierte Hygieneartikel kann aber durchaus, insbesondere entlang dieser Seitennahtbereiche, eine Sollbruchlinie, beispielsweise in Form eines Reißfadens aufweisen, um den Hygieneartikel ausgehend vom angelegten Zustand öffnen zu können. Auch können zusätzlich an sich beliebige Verschlusselemente, etwa klebende oder mechanisch haftende Verschlusslaschen, vorgesehen sein, um den Hygieneartikel nach dem Öffnen wieder wie eine Windel schließen zu können. Herstellerseitig ist der Hygieneartikel aber als Höschen ("pant") gefertigt und weist daher einen in Umfangsrichtung geschlossenen Hüftrand 20 auf, der eine allseits begrenzte Hüftöffnung bildet. Es handelt sich also um ein Höschen und nicht um eine öffen- und schließbare Windel.

Der Hygieneartikel 2 umfasst des Weiteren einen Absorptionskörper 22, der auch als funktionelle Einheit ausgebildet sein kann, d. h. eine flüssigkeitsabsorbierende Struktur mit einem flüssigkeitsundurchlässigen Backsheet und gegebenenfalls weiteren Komponenten, wie z. B. ein Topsheet und elastifizierte Bündchenelemente, aufweisen kann und als funktionelle Einheit auf Chassis bildende Materialien 24 des Hygieneartikels 2 aufgebracht werden kann. Diese Chassis bildenden Materialien 24 können beispielsweise an sich beliebige Backsheetmaterialien oder -verbundmaterialien darstellen. Es kommen in vorteilhafter Weise Vliesstoffe oder Vliesstofflaminate, aber auch Vliesfolienlaminate zum Einsatz, die eine textil anmutende Oberfläche aufweisen und vorzugsweise auch zumindest bereichsweise atmungsaktiv ausgebildet sind. Die Chassis bildenden Materialien 24 können auch eine körperzugewandte Topsheetlage umfassen, welche den Absorptionskörper 22 überfängt und sich in einer Querrichtung 26 und/oder in einer Längsrichtung 28 des Hygieneartikels 2 bis zu den Rändern desselben erstrecken kann.

Man erkennt in Figur 1 des Weiteren in Querrichtung 26 durchgehend erstreckte Elastifizierungsmittel 30, welche einen Hüftrandbereich 32 des Hygieneartikels 2 durchgehend elastifizieren. Des Weiteren sind im Rückenteil 4 und im Vorderteil 6 Elastifizierungsmittel 36 in einem Abstand 34 von 5 bis 15 mm voneinander durch unterbrochene Linien angedeutet, welche den Rückenteil 4 und den Vorderteil 6 nur bereichsweise elastifizieren und dazu beitragen, dass der Hygieneartikel 2 eine gute Passform erhält.

Man erkennt des Weiteren Elastifizierungsmittel 38, welche die Beinöffnungen 8 abschnittsweise elastifizieren, und zwar wie folgt:

Die Elastifizierungsmittel 38 erstrecken sich ausgehend vom Längsseitenrand 12 des Rückenteils 4 entlang eines Umfangs 40 der betrachteten Beinöffnung 8 in Richtung auf eine Schrittmittellinie 42. Die elastifizierende Wirkung der Elastifizierungsmittel 38 ist aber nicht durchgehend bis zur Schrittmittellinie 42, sondern sie ist ausgehend vom Längsseitenrand 12 auf einen ersten Umfangsabschnitt 44 der Beinöffnungen 8 beschränkt. Daran schließt sich ein nicht elastifizierter zweiter Umfangsabschnitt 46 der Beinöffnungen an und daran ein wiederum elastifizierter dritter Umfangsabschnitt 48. An diesen dritten Umfangsabschnitt 49 schließt sich ein vierter nicht elastifizierter Umfangsabschnitt an, der die Schrittmittellinie 42 überfängt. Der Umfang 40 der Beinöffnungen 8 ist daher nicht durchgehend elastifiziert, sondern im Rückenteil 4 und/oder im Vorderteil 6 in einem jeweiligen zweiten Umfangsabschnitt 46 und in einem vierten Umfangsabschnitt 49 nicht elastifiziert. Dies bedeutet, dass die Chassismaterialien 24 in diesem zweiten und vierten Umfangsabschnitt 46 und 49 nicht unter elastischer Vorspannung der zumeist fadenförmigen Elastifizierungsmittel 38 gegen die Hautoberfläche des Benutzers angelegt werden, sondern sanft gegen die Hautoberfläche anliegen. Es wird hierdurch eine Atmungsaktivität, d.h. eine bessere Belüftung der Bereiche außerhalb des Absorptionskörpers 22 erreicht, was durch den Pfeil 50 angedeutet ist. Außerdem wird in dem zweiten und vierten Umfangsabschnitt 46 und 49 keine Raffung oder Rüschung der Chassismaterialien 24 hervorgerufen, was zu einer Erhöhung der Hautfreundlichkeit führt, da es in geringerem Maß zu Hautreizungen kommt.

Die Darstellung der Elastifizierungsmittel 38 in Figur 1 ist derart, dass diese im zweiten Umfangsabschnitt 46 nicht vorgesehen sind. Dies kann in vorteilhafter Weise dadurch erreicht werden, dass die Elastifizierungsmittel 38 zwar durchgehend über alle drei Umfangsabschnitte zunächst erstreckt werden, dass sie aber nur im ersten und im dritten Umfangsabschnitt 44, 48 im vorgespannten Zustand mit den Chassismaterialien 24, vorzugsweise klebend verbunden werden. Die Elastifizierungsmittel 38 brauchen dann im zweiten Umfangsabschnitt 46 lediglich geschnitten zu werden und schnellen dann aufgrund ihrer Vorspannung bis zu ihrer Fixierung in dem ersten und dritten Umfangsabschnitt 44, 48 zurück, so dass der zweite Umfangsabschnitt 46 nicht mehr elastifiziert ist. Der vierte Umfangsabschnitt 49 ist dadurch nicht elastifiziert, dass die Elastifizierungsmittel 38 ausgehend von ihrer Erstreckung im dritten Umfangsabschnitt 46 in einer Kurve nach innen in die Querrichtung 26 verlaufen, so dass zwischen dem jeweiligen dritten Umfangsabschnitt 46 von Rückenteil 4 und Vorderteil 6 der vierte Umfangsabschnitt 49 gebildet wird.

In Figur 1 links sind im Vorderteil 6 die Elastifizierungsmittel 38 in dem zweiten Umfangsabschnitt 46 punktiert dargestellt. Hiermit soll eine weitere Ausführungsform der Erfindung verdeutlicht werden, wonach die zuvor vom Längsseitenrand 14 bis zur Schrittmittellinie 42 durchgehend erstreckten Elastifizierungsmittel 38 in dem zweiten Umfangsabschnitt 46 mehrfach im Abstand von 1 bis 3 mm geschnitten worden sind. Auf diese Weise wird eine Inaktivierung der Elastifizierungsmittel 38 in dem zweiten Umfangsabschnitt 46 erreicht. Eine solche mehrfache Durchtrennung der Elastifizierungsmittel 38 kann durch eine Messerwalze in einer Herstellungsmaschine erreicht werden, die über den in der Querrichtung 26 in Maschinenrichtung transportierten Flachmaterialien abrollt. Solchenfalls könnten die zuvor durchgehend aufgebrachten Elastifizierungsmittel 38 auch im zweiten Umfangsabschnitt 46 klebend mit den Chassismaterialien 24 verbunden werden, wenn dies herstellungstechnisch zweckmäßiger erscheint oder gewünscht sein sollte.

Nach einer weiteren bevorzugten Ausführungsform der Erfindung, die ebenfalls durch die unterbrochenen Linien in Figur 1 links im Vorderteil 6 angedeutet sein kann, sind die zuvor durchgehend von einem Längsseitenrand bis zur Schrittmittellinie 42 erstreckten Elastifizierungsmittel 38 in dem zweiten Umfangsabschnitt 46 durch Anwendung von Hitze und/oder Druck ihrer elastifizierenden Wirkung benommen. Durch Anwendung von Hitze und/oder Druck, also beispielsweise durch Ultraschallschweißverfahren oder durch rasterartig vorgesehene zu Erschmelzungen führende Wärmeeinleitung können die Elastifizierungsmittel 38 inaktiviert oder relaxiert werden. Sie werden dabei im Wesentlichen zerstört, so dass sie den zweiten Umfangsabschnitt 46 nicht mehr zu elastifizieren vermögen.

Außerdem ist im Bereich der Schrittmittellinie 42 eine in Querrichtung 26 erstreckte Elastifizierung vorgesehen. Dies ist - wie bereits vorausgehend angedeutet - in vorteilhafter Weise dadurch erreicht, dass die vorstehend genannten Elastifizierungsmittel 38 ausgehend von ihrer Erstreckung in Umfangsrichtung im dritten Umfangsabschnitt 48 nach innen "abbiegen", also in die Querrichtung 26 umgelenkt werden, und den Schrittbereich 10 in Querrichtung 26 unterhalb des Absorptionskörpers 22, also zwischen Absorptionskörper 22 und Chassismaterialien 24 traversieren, und dann wiederum stetig bzw. einstückig durchgehend in den Umfang der gegenüberliegenden Beinöffnung 8 sich weiter erstrecken. Für diese Führung der Elastifizierungsmittel 38 ausgehend vom dritten Umfangsabschnitt 46 wird selbständiger Schutz in Anspruch genommen, d.h. losgelöst von den weiteren Merkmalen der Ansprüche wird dies als selbständig erfindungsbegründend angesehen. Ausgehend von den vorausgehend beschriebenen Verfahren zur Relaxierung der Elastifizierungsmittel 28 in dem zweiten Umfangsabschnitt 46 ist es also möglich, die Elastifizierungsmittel 38 in der Maschinenrichtung, welche der Querrichtung 26 entspricht, durchgehend zu führen und mit den Chassismaterialien 24 des Hygieneartikels zu fügen, was als besonders vorteilhaft betrachtet wird.

Schließlich sei noch auf beidseits des Absorptionskörpers 22 vorgesehene elastifizierte und daher aufstehende Bündchenelemente 52 ("cuffs") hingewiesen, die eine seitliche Barriere für das Austreten von Körperausscheidungen bilden und auch flüssige Körperausscheidungen so lange im Bereich oberhalb des Absorptionskörpers 42 halten, bis diese durch den Absorptionskörper 22 aufgenommen sind.

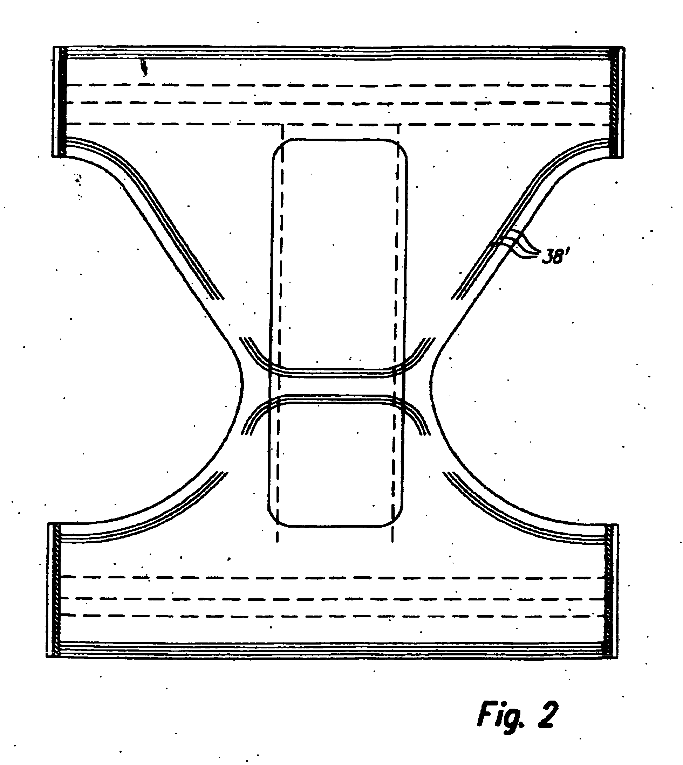

Gemäß Figur 2 ist eine weitere Ausführungsform angedeutet, bei der die Elastifizierungsmittel 38' zwar den Schrittbereich traversieren, jedoch anschließend in an sich beliebiger Weise inaktiviert wurden, d.h. sie wurden geschnitten oder in sonstiger Weise, insbesondere durch bereits erwähnte Anwendung von Hitze und/oder Druck ihrer elastifizierenden Wirkung benommen.

## Patentansprüche

1. Wegwerfbarer absorbierender Hygieneartikel (2) in Pantform, mit einem eine Hüftöffnung bildenden in Umfangsrichtung durchgehend geschlossenen Hüftrand (20) und mit Beinöffnungen (8), wobei der in Umfangsrichtung durchgehend geschlossene Hüftrand (20) und die Beinöffnungen (8) gebildet sind, indem Längsseitenrandabschnitte (16, 18) eines Vorderteils (6) und eines Rückenteils (4) herstellerseitig miteinander verbunden sind, und mit einem Absorptionskörper (22) und mit Elastifizierungsmitteln (38) zumindest im Bereich der Beinöffnungen (8)
**dadurch gekennzeichnet,**
**dass** im flachgelegten Zustand vor der herstellerseitigen Verbindung der Längsseitenrandabschnitte (16, 18) ausgehend von einem Längsseitenrand (14) des Vorderteils (6) des Artikels entlang eines Umfangs (40) einer jeweiligen Beinöffnung (8) in Richtung auf eine Schrittmittellinie (42) ein erster elastifizierter Umfangsabschnitt (44), daran anschließend ein zweiter nicht elastifizierter Umfangsabschnitt (46) und daran anschließend ein dritter elastifizierter Umfangsabschnitt (48) vorgesehen sind, dass im wesentlichen in einer Querrichtung (26) erstreckte Elastifizierungsmittel im Schrittbereich vorgesehen sind und dass die Elastifizierungsmittel (38) des dritten Umfangsabschnitts (48) in die Elastifizierungsmittel des Schrittbereichs übergehen.

2. Hygieneartikel nach Anspruch 1, **dadurch gekennzeichnet, dass** der nicht elastifizierte zweite Umfangsabschnitt (46) **dadurch** erhalten ist, dass dort zuvor erstreckte Elastifizierungsmittel (38) durchtrennt worden sind.

3. Hygieneartikel nach Anspruch 2, **dadurch gekennzeichnet, dass** die Durchtrennung durch eine Vielzahl von Schnitten erzielt ist.

4. Hygieneartikel nach Anspruch 1, **dadurch gekennzeichnet, dass** der nicht elastifizierte zweite Umfangsabschnitt (46) **dadurch** erhalten ist, dass dort zuvor erstreckte Elastifizierungsmittel (38) durch Anwendung von Hitze und/oder Druck und/oder Lasereinwirkung ihrer elastifizierenden Wirkung benommen worden sind.

5. Hygieneartikel nach Anspruch 4, **dadurch gekennzeichnet, dass** der zweite Umfangsabschnitt (46) im Bereich der dort zuvor erstreckten Elastifizierungsmittel (38) mittels Ultraschall beaufschlagt worden ist.

6. Hygieneartikel nach einem oder mehreren der vorstehenden Ansprüche, **dadurch gekennzeichnet, dass** im zweiten Umfangsabschnitt (46) makroskopische Belüftungsöffnungen in chassisbildenden Materialien vorgesehen sind.

7. Hygieneartikel nach einem oder mehreren der vorstehenden Ansprüche, **dadurch gekennzeichnet, dass** eine in Querrichtung (26) durchgehende Spur einer Anwendung von Hitze und/oder Druck vorgesehen ist, welche auch den zweiten Umfangsabschnitt (46) bei der jeweiligen Beinöffnung (8) überfängt.

8. Hygieneartikel nach einem oder mehreren der vorstehenden Ansprüche, **dadurch gekennzeichnet, dass** der Absorptionskörper (22) eine funktionelle Einheit mit einer absorbierenden Struktur und einem flüssigkeitsundurchlässigen Backsheet umfasst, die mit Chassismaterialien (24) des Artikels verbunden ist.

9. Hygieneartikel nach einem oder mehreren der vorstehenden Ansprüche, **dadurch gekennzeichnet, dass** die Chassismaterialien (24) aus Vliesstoff oder einem Vliesstofflaminat bestehen.

10. Hygieneartikel nach einem oder mehreren der vorstehenden Ansprüche, **dadurch gekennzeichnet, dass** die Erstreckung des ersten, elastifizierten Umfangsabschnitts (44) in Umfangsrichtung 3-15 cm, insbesondere 4-15 cm, insbesondere 5-15 cm, insbesondere 5 - 13 cm beträgt.

11. Hygieneartikel nach einem oder mehreren der vorstehenden Ansprüche, **dadurch gekennzeichnet, dass** die Erstreckung des zweiten, nicht plastifizierten Umfangsabschnitts (46) in Umfangsrichtung 1-10 cm, insbesondere 2-8 cm, insbesondere 3 - 8 cm beträgt.

12. Hygieneartikel nach einem oder mehreren der vorstehenden Ansprüche, **dadurch gekennzeichnet, dass** die Erstreckung des dritten, elastifizierten Umfangsabschnitts (48) in Umfangsrichtung 1 - 8 cm, insbesondere 1-7 cm, insbesondere 1 - 5 cm beträgt.

## Claims

1. Pants-shaped disposable absorbent hygiene article (2) with a hip edge (20) forming a hip opening which is continuously closed in the peripheral direction, and with leg openings (8), wherein the hip edge (20) which is continuously closed in the peripheral direction and the leg openings (8) are formed by connecting longitudinal side edge sections (16, 18) of a front part (6) and rear part (4) to each other at the manufacturing plant, and with an absorption body (22) and elasticising means (38) at least in the area of the leg openings (8), **characterized in that**, in the flat state before connecting the longitudinal side edge sections (16, 18) at the manufacturing plant, starting from a longitudinal side edge (14) of the front part (6) of the article along a periphery (40) of a respective leg opening (8) towards a center crotch line (42) there is provided a first elasticised peripheral section (44), followed by an adjacent second non-elasticised peripheral section (46), followed by an adjacent third elasticised peripheral section (48) and that elasticising means are provided in the crotch area which extend substantially in a transverse direction (26) and that the elasticising means (38) of the third peripheral section (48) merge into the elasticising means of the crotch area.

2. Hygiene article according to claim 1, **characterized in that** the non-elasticised second peripheral section (46) is obtained by cutting previously extending elasticising means (38).

3. Hygiene article according to claim 2, **characterized in that** cutting is performed by a plurality of cuts.

4. Hygiene article according to claim 1, **characterized in that** the non-elasticised second peripheral section (46) is obtained **in that** the elasticising effect of previously extending elasticising means (38) is eliminated through application of heat and/or pressure and/or laser radiation.

5. Hygiene article according to claim 4, **characterized in that** the second peripheral section (46) is acted upon by ultrasound in the area of the previously extending elasticising means (38).

6. Hygiene article according to any one or more of the preceding claims, **characterized in that** macroscopic ventilation openings are provided in the chassis-forming materials in the second peripheral section (46).

7. Hygiene article according to any one or more of the preceding claims, **characterized in that** a track of applying heat and/or pressure is provided which is continuous in the transverse direction (26), which also covers the second peripheral section (46) of the respective leg opening (8).

8. Hygiene article according to any one or more of the preceding claims, **characterized in that** the absorption body (22) comprises a functional unit with an absorbent structure and a liquid-impermeable backsheet which is connected to chassis materials (24) of the article.

9. Hygiene article according to any one or more of the preceding claims, **characterized in that** the chassis materials (24) consist of a non-woven material or a non-woven laminate.

10. Hygiene article according to any one or more of the preceding claims, **characterized in that** the first elasticising peripheral section (44) extends in the peripheral direction by 3 to 15 cm, in particular 4 to 15 cm, in particular 5 to 15 cm, in particular 5 to 13 cm.

11. Hygiene article according to any one or more of the preceding claims, **characterized in that** the second, non-elasticised peripheral section (46) extends in the peripheral direction by 1 to 10 cm, in particular 2 to 8 cm, in particular 3 to 8 cm.

12. Hygiene article according to any one or more of the preceding claims, **characterized in that** the third, elasticised peripheral section (48) extends in the peripheral direction by 1 to 8 cm, in particular 1 to 7 cm, in particular 1 to 5 cm.

## Revendications

1. Article d'hygiène absorbant jetable (2) se présentant sous la forme d'une culotte comprenant un bord de hanche (20) fermé de façon continue dans le sens de la circonférence pour former une ouverture de hanche et comprenant des ouvertures de jambes (8), le bord de hanche (20) fermé de façon continue dans le sens de la circonférence et les ouvertures de jambes (8) étant formés par la liaison en usine entre elles des sections de bord latéral longitudinal (16, 18) d'une partie avant (6) et d'une partie dorsale (4), et comprenant un corps absorbant (22) et des moyens d'élastification (38) au moins dans la zone des ouvertures de jambes (8), **caractérisé en ce que**, à l'état déplié avant la liaison en usine des sections de bord latéral longitudinal (16, 18), il est prévu, à partir d'un bord latéral longitudinal (14) de la partie avant (6) de l'article le long d'une circonférence (40) d'une ouverture de jambe (8) en direction d'une ligne médiane d'entrejambe (42) une première section de circonférence élastifiée (44), suivie d'une deuxième section de circonférence non élastifiée (46) et enfin une troisième section de circonférence élastifiée (48), **en ce qu'**il est prévu dans la section d'entrejambe des moyens d'élastification s'étendant essentiellement dans le sens transversal (26) et **en ce que** les moyens d'élastification (38) de la troisième section de circonférence (48) se transforment en les moyens d'élastification de la section d'entrejambe.

2. Article d'hygiène selon la revendication 1, **caractérisé en ce que** la deuxième section de circonférence non élastifiée (46) est obtenue en coupant les moyens d'élastification (38) qui s'y étendaient préalablement.

3. Article d'hygiène selon la revendication 2, **caractérisé en ce que** la séparation est obtenue par une pluralité de coupes.

4. Article d'hygiène selon la revendication 1, **caractérisé en ce que** la deuxième section de circonférence non élastifiée (46) est obtenue en retirant aux moyens d'élastification (38), qui s'y étendaient préalablement, leur action d'élastification, par application de chaleur et/ou de pression et/ou par effet de laser.

5. Article d'hygiène selon la revendication 4, **caractérisé en ce que** la deuxième section de circonférence (46) est sollicitée dans la zone des moyens d'élastification (38) qui s'étendaient préalablement au moyen d'ultrasons.

6. Article d'hygiène selon une ou plusieurs des revendications précédentes, **caractérisé en ce qu'**il est prévu dans la deuxième section de circonférence (46) des ouvertures d'aération macroscopiques dans les matériaux formant support.

7. Article d'hygiène selon une ou plusieurs des revendications précédentes, **caractérisé en ce qu'**il est prévu un marquage continu dans le sens transversal (26) par application de chaleur et/ou de pression, lequel recouvre également la deuxième section de circonférence (46) au niveau de l'ouverture de jambe (8) correspondante.

8. Article d'hygiène selon une ou plusieurs des revendications précédentes, **caractérisé en ce que** le corps absorbant (22) comprend une unité fonctionnelle avec une structure absorbante et une feuille arrière imperméable aux liquides, laquelle unité est reliée aux matériaux formant support (24) de l'article.

9. Article d'hygiène selon une ou plusieurs des revendications précédentes, **caractérisé en ce que** les matériaux formant support (24) se composent de non-tissé ou d'un stratifié de non-tissé.

10. Article d'hygiène selon une ou plusieurs des revendications précédentes, **caractérisé en ce que** l'étendue de la première section de circonférence élastifiée (44) dans le sens de la circonférence est comprise entre 3 et 15 cm, en particulier entre 4 et 15 cm, en particulier entre 5 et 15 cm et en particulier entre 5 et 13 cm.

11. Article d'hygiène selon une ou plusieurs des revendications précédentes, **caractérisé en ce que** l'étendue de la deuxième section de circonférence non élastifiée (46) dans le sens de la circonférence est comprise entre 1 et 10 cm, en particulier entre 2 et 8 cm et en particulier entre 3 et 8 cm.

12. Article d'hygiène selon une ou plusieurs des revendications précédentes, **caractérisé en ce que** l'étendue de la troisième section de circonférence élastifiée (48) dans le sens de la circonférence est comprise entre 1 et 8 cm, en particulier entre 1 et 7 cm et en particulier entre 1 et 5 cm.
